# EUROPÄISCHE PATENTSCHRIFT

(11) **EP 4 000 413 B1**
(45) Veröffentlichungstag und Bekanntmachung des Hinweises auf die Patenterteilung: **12.02.2025**
(21) Anmeldenummer: 20209032.0
(22) Anmeldetag: 20.11.2020
(51) Int. Cl.: A23L 2/44, A23L 29/00, A23L 33/105, C07C 51/00, C07C 51/47, C07C 51/48, C11B 5/00, C12N 9/18, A61K 36/22, C07C 51/42

(54) **VERFAHREN ZUR HERSTELLUNG EINES AUS SUMACH HERGESTELLTEN EXTRAKTS ENTHALTEND GALLUSSÄURE, DARAUS HERGESTELLTES WÄSSRIGES KONZENTRAT SOWIE LEBENSMITTEL UND NAHRUNGSERGÄNZUNGSMITTEL ENTHALTEND DAS WÄSSRIGE KONZENTRAT**
METHOD FOR PRODUCING AN EXTRACT OF SUMAC COMPRISING GALLIC ACID, AQUEOUS CONCENTRATE THEREOF, AND FOOD AND FOOD SUPPLEMENT COMPRISING THE AQUEOUS CONCENTRATE
PROCÉDÉ DE PRODUCTION D'UN EXTRAIT DE SUMAC COMPRENANT DE L'ACIDE GALLIQUE, SON CONCENTRÉ AQUEUX, ET ALIMENT ET COMPLÉMENT ALIMENTAIRE COMPRENANT LE CONCENTRÉ AQUEUX

(43) Veröffentlichungstag der Anmeldung: 25.05.2022
(73) Patentinhaber: Red Bull GmbH, 5330 Fuschl am See (AT)
(72) Erfinder: Nachbagauer, Josef, 5330 Fuschl am See (AT); Jenny, Marcel, 5330 Fuschl am See (AT); Rinderer, Christian, 5330 Fuschl am See (AT); Winter, Isabella, 5330 Fuschl am See (AT)
(74) Vertreter: Metten, Karl-Heinz

(56) Entgegenhaltungen:
- EP-A1- 2 022 346
- EP-A1- 2 606 902
- WO-A1-00/15044
- JP-B2- 4 630 295
- KAR B ET AL: "MICROBIAL PRODUCTION OF GALLIC ACID BY MODIFIED SOLID STATE FERMENTATION", JOURNAL OF INDUSTRIAL MICROBIOLOGY & BIOTECHNOLOGY, BASINGSTOKE, GB, vol. 23, no. 3, September 1999 (1999-09-01), pages 173 - 177, XP009017209, ISSN: 1367-5435, DOI: 10.1038/SJ.JIM.2900713
- POURRAT H ET AL: "MICROBIOLOGICAL PRODUCTION OF GALLIC ACID FROM RHUS CORIARIA L", BIOTECHNOLOGY LETTERS, vol. 9, no. 10, 1987, Dordrecht, pages 731 - 734, XP055794342, ISSN: 0141-5492
- ANONYMOUS: "Tannic acid", FNP 49, 1992, XP055794461, Retrieved from the Internet <URL:http://www.fao.org/fileadmin/user_upload/jecfa_additives/docs/Monograph1/Additive-454.pdf> [retrieved on 20210412]
- MARTINS ISABELA M ET AL: "Tannase enhances the anti-inflammatory effect of grape pomace in Caco-2 cells treated with IL-1[beta]", JOURNAL OF FUNCTIONAL FOODS, ELSEVIER BV, NL, vol. 29, 19 December 2016 (2016-12-19), pages 69 - 76, XP029886766, ISSN: 1756-4646, DOI: 10.1016/J.JFF.2016.12.011
- BURSAL ERCAN ET AL: "Evaluation of reducing power and radical scavenging activities of water and ethanol extracts from sumac (Rhus coriaria L.)", FOOD RESEARCH INTERNATIONAL, vol. 44, no. 7, 1 August 2011 (2011-08-01), AMSTERDAM, NL, pages 2217 - 2221, XP093071172, ISSN: 0963-9969, DOI: 10.1016/j.foodres.2010.11.001

## Beschreibung

Die vorliegende Erfindung betrifft ein Verfahren zur Herstellung eines Extrakts enthaltend Gallussäure, insbesondere in hohen Konzentrationen. Ferner betrifft die Erfindung ein wässriges Konzentrat enthaltend Gallussäure. Außerdem betrifft die Erfindung Lebensmittel und Nahrungsergänzungsmittel enthaltend das erfindungsgemäße wässrige Konzentrat. Gallussäure (3,4,5-Trihydroxybenzoesäure) ist Säurekomponente der Gallotannine, bei denen es sich um hydrolysierbare Gerbstoffe handelt. Sie kommen z.B. in der Eichenrinde und in Galläpfeln vor. Gallussäure wird für die Herstellung von Antioxidantien, Sonnenschutzmitteln und Farbstoffen eingesetzt. Des Weiteren wurde postuliert, dass die Gallussäure aufgrund ihrer hypoglykämischen Eigenschaften bei adipösen Personen, die gallussäurereiche Nahrungsmittel zu sich nehmen, nachteilige gesundheitliche Auswirkungen verhindern helfen soll. Mit dem Verzehr von Gallussäure sollen ein nachweisbarer DNA-Schutz bzw. eine verringerte Oxidation von DNA-Basen sowie reduzierte Entzündungsparameter einhergehen (s.a. T. Setayesh et al. "Gallic acid, a common dietary phenolic protects against high fat diet induced DNA damage" in European Journal of Nutrition, Vol. 58, Seiten 2315 bis 26, 2019).

Gemäß der EP 1 942 919 B1 soll eine Zusammensetzung, enthaltend einen Polyphenolextrakt aus Trauben, der 400 bis 1500 ppm Gallussäure aufweisen kann, zur Behandlung von Metabolischem Syndrom oder Prähypertonie geeignet sein.

Gemäß der EP 2 095 718 B1 soll sich ein Teegetränk, dass auf einen Tee-Extrakt zurückgeht, in dem das Gewichtsverhältnis von Gallussäure zu nicht-polymeren Catechinen nicht größer als 0,3 sein darf, durch eine verringerte Bitterkeit und einen reduzierten sauren Geschmack auszeichnen.

In der EP 2 143 344 B1 wird eine Getränkezusammensetzung beschrieben, enthaltend 0,5 bis 25,0 Gew.% nicht-polymere Catechine, ein Kohlenhydrat und eine Hydroxycarbonsäure. In diesen Zusammensetzungen hat der Gehalt an Gallussäure niedriger als 0,6 Gew.% zu sein.

B. Kar et al. beschreiben in dem Artikel "Microbial production of gallic acid by modified solid state fermentation", Journal of Industrial Microbiology & Biotechnology, 1999, 23, Seiten 173-177, die Biokonversion von Tannin zu Gallussäure mit Hilfe des Enzyms Tannase. Diethylether wurde als Lösungsmittel für die Extraktion von Gallussäure aus der fermentierten Biomasse verwendet.

H. Purrat et al. beschreiben in der Publikation "Microbial production of gallic acid form Rhus Coriaria L.", Biotechnology Letters Vol 9, No 10, 1987, Seiten 731-734, die Herstellung von Gallussäure-haltigen Extrakten aus Sumachblättern, wobei diese Sumachblätter zunächst mit siedendem Wasser versetzt und anschließend, ohne vorherige Filtration, in Gegenwart von *A. niger*, einem Tannase-produzierendem Stamm, fermentativ behandelt werden. Aus dem abfiltrierten Überstand wurde kristalline Gallussäure gewonnen.

Gemäß der Publikation *"*Tannic acid", erstellt auf dem 39. Joint Expert Committee on Food Additives (JECFA), 1992, und veröffentlicht in FNP Add 1 (1992), kann Tanninsäure, welche gut in Wasser und Ethanol löslich ist, durch Lösungsmittelextraktion aus z.B. Sumachgewächsen gewonnen und zu Gallussäure hydrolysiert werden.

Die JP 4630295 B2 offenbart ein Verfahren zur Herstellung eines Tee-Extrakts, bei dem Tee-Rohmaterialien in einem ersten Schritt in Wasser mit Tannase behandelt werden. Anschließend soll nach Zugabe einer wässrigen Ethanollösung die Extraktion vonstattengehen. Die Extraktion in Anwesenheit von Ethanol soll die Ausbeute an Catechinen erhöhen.

Die EP 2 022 346 A1 beschreibt die Behandlung von Grünteeextrakten mit hohem Catechingehalt mit Tannase sowie deren anschließende Sprühtrocknung. Das erhaltene Pulver wurde mit einem Lösungsmittelgemisch aus Ethanol und Wasser (85:15) extrahiert und sodann mit Aktivkohle gereinigt.

Aus der WO 00/150 044 A1 geht eine pharmazeutische Zusammensetzung hervor, enthaltend eine wirksame Menge Tannin, Gallussäure und/oder Ellagsäure.

In der EP 2 606 902 A1 wird ein Verfahren zur Herstellung eines Extrakts aus *Rhus copallina* beschrieben, bei dem zerkleinerte Blätter von *Rhus copallina L.* mit einem Gemisch aus Wasser und Ethanol extrahiert werden. Die Extraktion kann beispielsweise mittels Mazeration oder Perkolation erfolgen.

I. Martins et al. beschreiben in dem Artikel "Tannase enhances the anti-inflammatory effect of grape pomace in Caco-2 cells treated with IL-1β", Journal of Functional Foods 29 (2017), Seiten 69-76, die Extraktion von Traubenkerntrester mit einer wässrigen Tannaselösung. Nach Versetzen mit Methanol, Ultraschallbehandlung, Abzentrifugieren und Gefriertrocknung des Überstands erhielt man einen Feststoff enthaltend in geringen Mengen Gallussäure.

E. Bursal et al. berichten in "Evaluation of reducing power and radical scavenging activities of water and ethanol extracts from suma (Rhus coriaria L.)", Food Research International 44 (2011), Seiten 2217-2221, dass die Extraktion von Sumachpflanzen mit Ethanol im Vergleich zu einer Extraktion mit Wasser nicht zu einer verbesserten Extraktion phenolischer Verbindungen führt.

Gallussäure wird bislang häufig mithilfe von wässrigen Extraktionsverfahren gewonnen. Diese Verfahren führen jedoch regelmäßig nur zu geringen Ausbeuten an Gallussäure und sind material-, apparate- und kostenintensiv.

Der vorliegenden Erfindung lag daher die Aufgabe zugrunde, ein Verfahren zur Gewinnung von Gallussäure verfügbar zu machen, das nicht mit den Nachteilen des Stands der Technik behaftet ist und das insbesondere Gallussäure verlässlich in guten Ausbeuten auf effiziente Weise verfügbar macht.

Demgemäß wurde ein Verfahren zur Herstellung eines Extrakts enthaltend Gallussäure gefunden (auch erste Ausführungsvariante des erfindungsgemäßen Verfahrens genannt), umfassend die Schritte
a) Zurverfügungstellung von, insbesondere zerkleinerten und/oder getrockneten, Pflanzen oder Pflanzenbestandteilen, enthaltend Tannine, insbesondere Tanninsäure, wobei die zur Verfügung gestellte Pflanze ein Sumachgewächs umfasst oder darstellt und wobei die Pflanzenbestandteile Blätter, Hölzer, Rinde, Samen, Wurzeln und/oder Früchte von Sumachgewächsen umfassen oder darstellen,
b) Zurverfügungstellung des Enzyms Tannase (IUB 3.1.1.20),
c) Zurverfügungstellung von Ethanol,
d) gegebenenfalls Zerkleinern bzw. weiteres Zerkleinern der Pflanzen oder Pflanzenbestandteile gemäß a) und
e) extrahierendes Behandeln der gemäß Schritt a) und/oder d) erhaltenen, insbesondere zerkleinerten, Pflanzen oder Pflanzenbestandteile mit Wasser in Gegenwart des Enzyms Tannase (IUB 3.1.1.20) unter Erhalt eines Produktgemisches enthaltend Gallussäure,
f) das Abtrennen der, insbesondere zerkleinerten, Pflanzen oder Pflanzenbestandteile aus dem gemäß Schritt e) erhaltenen Produktgemisches, insbesondere im Anschluss an Schritt e), und
g) das evaporative Konzentrieren des gemäß Schritt e) oder f) erhaltenen Produktgemisches, enthaltend Gallussäure, insbesondere im Anschluss an Schritt e) oder f) und/oder vor Schritt h), und
h) Behandeln des Produktgemisches gemäß Schritt e) oder f) mit Ethanol und Behandeln des Produktgemisches gemäß Schritt e) oder f) oder g) mit Wärme und
i) Filtrieren des behandelten Produktgemisches gemäß Schritt h) und
j) Entfernen des in Schritt h) zugegebenen Ethanols aus dem behandelten Produktgemisch gemäß Schritt h) oder dem filtrierten Produktgemisch gemäß Schritt i),
k) evaporatives Konzentrieren des nach Schritt j) erhaltenen wässrigen Extrakts, enthaltend Gallussäure unter Erhalt eines wässrigen Konzentrats,
wobei in Schritt h) Ethanol in einer Menge im Bereich von 20 bis 65 Gewichtsprozent, bezogen auf das Gesamtgewicht aus dem Ethanol und dem Wasser bzw. dem wässrigen System zugegeben wird.

Bei den, insbesondere zerkleinerten und/oder getrockneten, Pflanzen oder Pflanzenbestandteilen wird zurückgegriffen auf Blätter, Hölzer, Rinden, Früchte, Same und/oder Wurzeln, vorzugsweise den Früchten, von Sumachgewächsen (*Rhus*), vor allem auf die Blätter, Rinde und Früchte von Gewürzsumach (*Rhus coriaria*), insbesondere die Früchte.

Als, insbesondere zerkleinerte und/oder getrocknete, Pflanzen kommen Sumachgewächse (*Rhus*) zum Einsatz, wobei insbesondere zurückgegriffen wird auf Gewürzsumach (*Rhus coriaria*). Bevorzugte Pflanzenbestandteile von Sumachgewächsen, insbesondere von Gewürzsumach (*Rhus coriaria*), umfassen Blätter, Rinde und/oder Früchte, wobei die Früchte wiederum besonders bevorzugt sind.

Das erfindungsgemäße Verfahren zeichnet sich in einer bevorzugten Ausführungsform auch dadurch aus, dass das extrahierende Behandeln gemäß Schritt e) sich über eine Zeitdauer von maximal 4 Stunden, insbesondere über eine Zeitdauer von maximal 3 Stunden, erstreckt. Mit dem erfindungsgemäßen Verfahren gemäß der ersten Ausführungsvariante kann demgemäß ein Extrakt mit hoher Konzentration an Gallussäure schon innerhalb relativ kurzer Extraktionszeiten erhalten werden.

Das erfindungsgemäße Verfahren gemäß der ersten Ausführungsvariante umfasst ferner (Schritt f)) das Abtrennen der, insbesondere zerkleinerten, Pflanzen oder Pflanzenbestandteile aus dem gemäß Schritt e) erhaltenen Produktgemisch, insbesondere im Anschluss an Schritt e), vorzugsweise mit Hilfe eines Siebs.

Zusätzlich umfasst das erfindungsgemäße Verfahren gemäß der ersten Ausführungsvariante außerdem (Schritt g)) das evaporative Konzentrieren des gemäß Schritt e) oder f) erhaltenen Produktgemisches, enthaltend Gallussäure, insbesondere im Anschluss an Schritt e) oder f) und/oder vor Schritt h).

Auch umfasst das erfindungsgemäße Verfahren gemäß der ersten Ausführungsvariante ferner zusätzlich (Schritt h)) das Behandeln des Produktgemisches gemäß Schritt e) oder f) mit Ethanol und das Behandeln des Produktgemisches gemäß Schritt e) bzw. f) oder g) mit Wärme. Durch die Behandlung mit dem Ethanol und zusätzlich durch die Behandlung mit Wärme gelingt regelmäßig eine Deaktivierung des Enzyms Tannase. Dies erfolgt insbesondere auch dadurch, dass man das Produktgemisch mit dem Ethanol in Schritt h) bei Temperaturen im Bereich von 40 bis 80 °C oder oberhalb von 45 bis 80 °C, bevorzugt 40 bis 75 °C oder 48 bis 75 °C und besonders bevorzugt 50 bis 70 °C behandelt. Zur weitergehenden Optimierung von Verfahrensschritt h) ist vorgesehen, dass in diesem Schritt zusätzlich das Ethanol in einer Menge im Bereich von 20 bis 65 Gewichtsprozent, bevorzugt im Bereich von 25 bis 60 und insbesondere im Bereich von 40 bis 60 Gewichtsprozent, jeweils bezogen auf das Gesamtgewicht aus dem Ethanol und dem Wasser bzw. dem wässrigen System, zugegeben wird. Mit Schritt h), insbesondere dem Behandeln mit Ethanol gelingt es regelmäßig sowohl, das Enzym zu deaktivieren, wie auch Phytoproteine und/oder Polysaccharide zu denaturieren und auszufällen.

Die Schritte f) und g) folgen aufeinander. In einer besonders bevorzugten Ausführungsform schließen sich an den Verfahrensschritt e) die Verfahrensschritte f), g) h) in dieser Reihenfolge an.

Bei dem erfindungsgemäßen Verfahren gemäß der ersten Ausführungsvariante filtriert man das behandelte Produktgemisch gemäß Schritt h), beispielsweise mit einem Filter mit einer Maschenweite im Bereich von 0,10 bis 1,0 µm, bevorzugt im Bereich von 0,25 µm bis 1,0 µm und besonders bevorzugt im Bereich von 0,35 µm bis 0,55 µm. Auf diese Weise erhält man im Allgemeinen einen klaren Extrakt.

Das in Schritt h) zugegebene Ethanol wird anschließend wieder aus dem behandelten Produktgemisch gemäß Schritt h) oder dem filtrierten Produktgemisch gemäß Schritt i) entfernt (Schritt j). Die extrahierende Behandlung gemäß Schritt e) wird bevorzugt bei Temperaturen im Bereich von 20 bis 45 °C oder 30 bis 43 °C durchgeführt, wobei Temperaturen im Bereich von 35 bis 40 °C, insbesondere im Bereich von 36 bis 38 °C, besonders bevorzugt sind.

Die der Erfindung zugrunde liegende Aufgabe wird des Weiteren gelöst durch ein Verfahren zur Herstellung eines Extrakts enthaltend Gallussäure (auch zweite Ausführungsvariante des erfindungsgemäßen Verfahrens genannt), umfassend die Schritte
1) Zurverfügungstellung von, insbesondere zerkleinerten und/oder getrockneten, Pflanzen oder Pflanzenbestandteilen, enthaltend Tannine, insbesondere Tanninsäure, wobei die zur Verfügung gestellte Pflanze ein Sumachgewächs umfasst oder darstellt und wobei die Pflanzenbestandteile Blätter, Hölzer, Rinde, Samen, Wurzeln und/oder Früchte von Sumachgewächsen umfassen oder darstellen,
2) Zurverfügungstellung des Enzyms Tannase (IUB 3.1.1.20),
3) Zurverfügungstellung von Ethanol,
4) gegebenenfalls Zerkleinern bzw. weiteres Zerkleinern der Pflanzen oder Pflanzenbestandteile gemäß Schritt 1),
5) extrahierendes Behandeln der, insbesondere getrockneten und/oder zerkleinerten, Pflanzen oder Pflanzenbestandteile gemäß Schritt 1) und/oder 4) mit einem Gemisch enthaltend das Ethanol und Wasser unter Erhalt eines Produktgemisches, enthaltend Polyphenole,
6) Abtrennen der, insbesondere zerkleinerten, Pflanzen oder Pflanzenbestandteile aus dem Produktgemisch gemäß Schritt 5) unter Erhalt eines Flüssigextrakts enthaltend die Polyphenole, insbesondere mit Hilfe eines Siebs,
7) Entfernen des Ethanols aus dem Flüssigextrakt gemäß Schritt 6) unter Erhalt eines wässrigen Extrakts enthaltend die Polyphenole,
8) Behandeln des wässrigen Extrakts gemäß Schritt 7) mit dem Enzym Tannase (IUB 3.1.1.20) unter Erhalt eines wässrigen Systems, enthaltend Gallussäure,
9) evaporatives Konzentrieren des wässrigen Systems, enthaltend Gallussäure, gemäß Schritt 8) unter Erhalt eines wässrigen Konzentrats,
wobei in Schritt 5) Ethanol in einer Menge im Bereich von 60 bis 90 Gewichtsprozent, bezogen auf das Gesamtgewicht aus Ethanol und dem Wasser, zugegeben wird.

Die der Erfindung zugrunde liegende Aufgabe wird mit dem Verfahren gemäß der zweiten Ausführungsvariante besonders gut auch dadurch gelöst, dass man das extrahierende Behandeln gemäß Schritt 5) bei Temperaturen im Bereich von 50 bis 70 °C, insbesondere im Bereich von 55 bis 65 °C, durchführt.

In einer bevorzugten Ausgestaltung zeichnet sich die zweiter Ausführungsvariante des erfindungsgemäßen Verfahrens auch dadurch aus, dass in Schritt 5) Ethanol in einer Menge im Bereich von 70 bis 85 Gewichtsprozent, bezogen auf das Gesamtgewicht aus Ethanol und dem Wasser, zugegeben wird.

Hierbei wird in der zweiten Ausführungsvariante des erfindungsgemäßen Verfahrens das Behandeln des wässrigen Extrakts gemäß Schritt 8) mit dem Enzym Tannase bevorzugt bei Temperaturen im Bereich von 20 bis 45 °C, bevorzugt 30 bis 43 °C, besonders bevorzugt 35 bis 40 °C und insbesondere im Bereich von 36 bis 38 °C, durchgeführt. Überraschend hat sich gezeigt, dass eine besonders effiziente Verfahrensführung auch dadurch gelingt, dass die, insbesondere gemahlenen, vorzugsweise zerkleinerten, Pflanzen oder Pflanzenbestandteile in Schritt e) gemäß der ersten Ausführungsvariante bzw. Schritt 5) gemäß der zweiten Ausführungsvariante immobilisiert, insbesondere in einem Siebträger immobilisiert, vorliegen.

Das extrahierende Behandeln gemäß Schritt e) in der ersten Ausführungsvariante bzw. Schritt 5) in der zweiten Ausführungsvariante umfasst bevorzugt ein Perkolationsverfahren oder stellt ein solches dar.

Besonders hohe Ausbeuten bzw. Konzentrationen an Gallussäure lassen sich mit den erfindungsgemäßen Verfahren auch dadurch erreichen, dass man die Menge an Tannase in Schritt e) gemäß der ersten Ausführungsvariante bzw. Schritt 8) gemäß der zweiten Ausführungsvariante im Bereich von 0,1 U/g bis 100,0 U/g, bevorzugt im Bereich von 0,2 U/g bis 50,0 U/g, bezogen auf die Menge an Pflanzen oder Pflanzenbestandteilen, einstellt. Erfindungsgemäß wird der nach Schritt j) erhaltene wässrige Extrakt, enthaltend Gallussäure, gemäß der ersten Ausführungsvariante bzw. das wässrige System, enthaltend Gallussäure, gemäß Schritt 8) gemäß der zweiten Ausführungsvariante evaporativ aufzukonzentriert unter Erhalt eines wässrigen Konzentrats. Hierbei handelt sich bevorzugt um einen sogenannten Spissumextrakt. Dieses wässrige Konzentrat bzw. dieser Spissumextrakt, erhalten aus Sumachgewächsen, besonders bevorzugt Gewürzsumach, oder Bestandteilen hiervon, insbesondere den Früchten, vor allem erhalten nach der ersten Ausführungsvariante, verfügt dabei insbesondere über einen Brix-Wert im Bereich von 1° bis 100 ° Brix, bevorzugt im Bereich von 10° bis 70° Brix und besonders bevorzugt im Bereich von 20° bis 50° Brix. Ganz besonders bevorzugt werden bei dem Schritt des Aufkonzentrierens Konzentrate mit Brix-Werten im Bereich von 20° bis 40 ° Brix, insbesondere im Bereich von 25° bis 35° Brix, angestrebt bzw. erhalten. Die Bestimmung von Grad Brix ist dem Fachmann geläufig. Eine Flüssigkeit weist einen Brix-Wert von 1° auf, wenn sie dieselbe Dichte hat wie eine Lösung von 1 g Saccharose in 100 g Saccharose/Wasser-Lösung, und einen Brix-Wert von 10°, wenn ihre Dichte die einer Lösung von 10 g Saccharose in 100 g Saccharose-Wasser-Lösung beträgt. Somit bedeutet die Angabe in Grad Brix, dass die Dichte der gemessenen Flüssigkeit der Dichte einer Lösung von Saccharose in Wasser entspricht, die so viele Gramm Saccharose pro 100 g Lösung enthält, wie die Gradangabe nennt.

Die der Erfindung zugrunde liegende Aufgabe wird des Weiteren gelöst durch ein wässriges Konzentrat gemäß Anspruch 18, enthaltend Gallussäure, erhalten nach der ersten oder zweiten Ausführungsvariante, wobei die für dieses wässrige Konzentrat verwendete Menge an Trockenmasse im Bereich von 5 bis 80 Gewichtsprozent, bevorzugt im Bereich von 10 bis 50 Gewichtsprozent und besonders bevorzugt im Bereich von 15 bis 40 Gewichtsprozent, jeweils bezogen auf das Gesamtgewicht des wässrigen Konzentrats, liegt. Diese wässrigen Konzentrate eignen sich insbesondere für die Herstellung von Lebensmitteln und Nahrungsergänzungsmitteln, insbesondere von solchen in Form von Getränken.

Unter Trockenmasse im Sinne der Erfindung soll diejenige Trockenmasse verstanden werden, die man durch Entfernen sämtlicher wässriger und nicht-wässriger Lösungsmittel aus den nach den erfindungsgemäßen Verfahren zugänglichen Extrakten erhält.

Die erfindungsgemäßen wässrigen Konzentrate werden entweder nach dem erfindungsgemäßen Verfahren gemäß der ersten Ausführungsvariante oder nach dem erfindungsgemäßen Verfahren gemäß der zweiten Ausführungsvariante erhalten, wobei diejenigen wässrigen Konzentrate bevorzugt sind, die nach dem erfindungsgemäßen Verfahren gemäß der ersten Ausführungsvariante zugänglich sind.

Die erfindungsgemäßen wässrigen Konzentrate, erhalten aus Sumachgewächsen, besonders bevorzugt Gewürzsumach, oder Bestandteilen hiervon, insbesondere den Früchten, insbesondere erhalten gemäß der ersten Ausführungsvariante, verfügen über einen pH-Wert im Bereich von 1,5 bis 5,5, bevorzugt im Bereich von 1,75 bis 4,5 und besonders bevorzugt im Bereich von 2,0 bis 4,0, bzw. sind auf einen pH-Wert im Bereich von 1,5 bis 5,5, bevorzugt im Bereich von 1,75 bis 4,5 und besonders bevorzugt im Bereich von 2,0 bis 4,0, eingestellt.

In den erfindungsgemäßen wässrigen Konzentraten, erhalten aus Sumachgewächsen, besonders bevorzugt Gewürzsumach, oder Bestandteilen hiervon, insbesondere den Früchten, insbesondere erhalten gemäß der ersten Ausführungsvariante, beträgt die Menge an Gallussäure mindestens 10 g/kg Trockenmasse, bevorzugt mindestens 50 g/kg Trockenmasse und besonders bevorzugt mindestens 75 g/kg Trockenmasse, und/oder Gallussäure liegt in einer Menge im Bereich von 10 bis 250 g/kg Trockenmasse, bevorzugt im Bereich von 50 bis 200 g/kg Trockenmasse und besonders bevorzugt im Bereich von 75 bis 175 g/kg Trockenmasse, vor, jeweils bezogen auf das Gesamtgewicht des wässrigen Konzentrats.

Die erfindungsgemäßen wässrigen Konzentrate, erhalten aus Sumachgewächsen, bevorzugt Gewürzsumach, oder Bestandteilen hiervon, insbesondere den Früchten, insbesondere erhalten gemäß der ersten Ausführungsvariante, verfügen über eine relative Dichte [20/20] im Bereich von 1,001 bis 1,4000, bevorzugt im Bereich von 1,010 bis 1,2500. Die Relative Dichte [20/20] ist der Quotient aus der Dichte der untersuchten Probe (gemessen bei 20°C) und der Dichte von Wasser bei 20°C. Die Messung erfolgt hierbei insbesondere mittels Biegeschwinger nach § 35 LMBG 36.0 3a (deutsches Lebensmittel- und Bedarfsgegenständegesetz in der zum Anmeldetag geltenden Fassung) (Bestimmung der relativen Dichte d 20/20 von Würze und Bier (Biegeschwinger-Verfahren). Besonders geeignete wässrige Konzentrate zeichnen sich durch einen Gesamtphenolgehalt im Bereich von 5 bis 350 g GAE/kg, bevorzugt im Bereich von 50 bis 300 g GAE/kg und besonders bevorzugt im Bereich von 75 bis 250 g GAE/kg sowie insbesondere im Bereich von 125 bis 200 g GAE/kg aus, jeweils bezogen auf die Trockenmasse des Extrakts.

Der Gesamtphenolgehalt kann hierbei nach der Methode gemäß DIN ISO 14502-1:2007-11 (Bestimmung von charakteristischen Substanzen von grünem und schwarzem Tee - Teil 1: Gesamt-Polyphenolgehalt in Tee - Colorimetrisches Verfahren mit Folin-Ciocalteu-Reagenz) ermittelt werden.

Außerdem wird die der Erfindung zugrunde liegende Aufgabe gelöst durch ein Lebensmittel, beispielsweise ein Getränk, wie auch durch ein Nahrungsergänzungsmittel, enthaltend oder gebildet aus dem erfindungsgemäßen wässrigen Konzentrat.

Mit der vorliegenden Erfindung geht die überraschende Erkenntnis einher, dass sich bei Einsatz der erfindungsgemäßen Extraktionsverfahren gemäß der aus ersten Ausführungsvariante wie auch gemäß der zweiten Ausführungsvariante, insbesondere jedoch gemäß der ersten Ausführungsvariante, Gallussäure in hohen Ausbeuten effektiv und kostengünstig aus vielfältigen pflanzlichen Materialien bzw. Bestandteilen hiervon gewinnen lässt, wodurch wässrige Konzentrate mit einer sehr hohen Gallussäure-Konzentration zugänglich sind. Hierbei wurde überraschend festgestellt, dass die erfindungsgemäßen wässrigen Konzentrate, erhalten aus Sumachgewächsen, besonders bevorzugt Gewürzsumach, oder Bestandteilen hiervon, insbesondere den Früchten, vor allem erhalten gemäß der ersten Ausführungsvariante, mit einer Trolox-äquivalenten antioxidativen Kapazität im Bereich von 500 bis 6000 µmol/g, bevorzugt im Bereich von 1000 bis 5000 µmol/g und besonders bevorzugt von 2000 bis 4000 µmol/g, jeweils bezogen auf die Trockenmasse des Extrakts, ausgestattet sind. Die Trolox-äquivalente antioxidative Kapazität kann zweckmäßigerweise mit dem DPPH (2,2-diphenyl-1-picrylhydrazyl)-Assay bestimmt werden. Dieses Bestimmungsverfahren ist dem Fachmann geläufig und wird beispielsweise beschrieben von O. Sharma und T. N. Bhat in "DPPH antioxidant assay revisited", Food Chemistry 113(4):1202-1205, April 2009, sowie von A. Karioti et al. in "Composition and antioxidant activity of the essential oils of Xylopia aethiopica (Dun) A. Rich. (Annonaceae) leaves, stem bark, root bark, and fresh and dried fruits, growing in Ghana", J. Agric. Food Chem. 29;52(26):8094-8, December 2004. Beispielsweise können bei diesem Verfahren 10 mg des stabilen Radikals DPPH in 100 ml Methanol gelöst und nach Mischen mit dem jeweiligen Probenextrakt bzw. dem Standard (Trolox) für 30 min bei Raumtemperatur inkubiert sowie anschließend die Extinktion photometrisch bei 517 nm bestimmt werden. Das antioxidative Potential der Proben kann dann aus einer linearen Beziehung zwischen Absorption und den Standardkonzentrationen bestimmt werden. Die Ergebnisse lassen sich als Troloxäquivalente bzw. als Mikromol Trolox/g Probe berechnen sowie in Bezug zur Trockenmasse setzen. Mit dem beschriebenen Verfahren wird eine Extinktions-Differenz ermittelt, aus der durch Vergleich mit der durch Trolox in verschiedenen Konzentrationen verursachten Minderung der Extinktion die antioxidative Kapazität der untersuchten Substanz ermittelt werden kann.

Die erfindungsgemäßen wässrigen Konzentrate können unmittelbar für die Herstellung von Lebensmitteln, insbesondere Getränken, und Nahrungsergänzungsmittel verwendet werden.Die in der vorstehenden Beschreibung und in den Ansprüchen offenbarten Merkmale der Erfindung können sowohl einzeln aus auch in jeder beliebigen Kombination für die Verwirklichung der Erfindung in ihren verschiedenen Ausführungsformen wesentlich sein.

## Patentansprüche

1. Verfahren zur Herstellung eines Extrakts enthaltend Gallussäure, umfassend die Schritte
a) Zurverfügungstellung von, insbesondere getrockneten und/oder, insbesondere und, zerkleinerten, Pflanzen oder Pflanzenbestandteilen, enthaltend Tannine, insbesondere Tanninsäure, wobei die zur Verfügung gestellte Pflanze ein Sumachgewächs umfasst oder darstellt und wobei die Pflanzenbestandteile Blätter, Hölzer, Rinde, Samen, Wurzeln und/oder Früchte von Sumachgewächsen umfassen oder darstellen,
b) Zurverfügungstellung des Enzyms Tannase,
c) Zurverfügungstellung von Ethanol,
d) gegebenenfalls Zerkleinern bzw. weiteres Zerkleinern der Pflanzen oder Pflanzenbestandteile gemäß Schritt a),
e) extrahierendes Behandeln der gemäß Schritt a) und/oder d), insbesondere oder, erhaltenen, insbesondere zerkleinerten, Pflanzen oder Pflanzenbestandteile mit Wasser in Gegenwart des Enzyms Tannase unter Erhalt eines Produktgemisches enthaltend Gallussäure,
f) das Abtrennen der, insbesondere zerkleinerten, Pflanzen oder Pflanzenbestandteile aus dem gemäß Schritt e) erhaltenen Produktgemisches, insbesondere im Anschluss an Schritt e), und,
g) das evaporative Konzentrieren des gemäß Schritt e) oder f) erhaltenen Produktgemisches, enthaltend Gallussäure, insbesondere im Anschluss an Schritt e) oder f) und/oder vor Schritt h), und
h) Behandeln des Produktgemisches gemäß Schritt e) oder f) mit Ethanol und Behandeln des Produktgemisches gemäß Schritt e) oder f) oder g) mit Wärme zur Deaktivierung des Enzyms Tannase und zur Denaturierung und Ausfällung von Proteinen und/oder Polysacchariden und
i) Filtrieren des behandelten Produktgemisches gemäß Schritt h) und
j) Entfernen des in Schritt h) zugegebenen Ethanols aus dem behandelten Produktgemisch gemäß Schritt h) oder dem filtrierten Produktgemisch gemäß Schritt i),
k) evaporatives Konzentrieren des nach Schritt j) erhaltenen wässrigen Extrakts, enthaltend Gallussäure unter Erhalt eines wässrigen Konzentrats,
wobei in Schritt h) Ethanol in einer Menge im Bereich von 20 bis 65 Gewichtsprozent, bezogen auf das Gesamtgewicht aus dem Ethanol und dem Wasser bzw. dem wässrigen System, zugegeben wird.

2. Verfahren nach Anspruch 1, **dadurch gekennzeichnet, dass**
das extrahierende Behandeln gemäß Schritt e) bei Temperaturen im Bereich von 20 bis 45 °C durchgeführt wird.

3. Verfahren nach einem der vorangehenden Ansprüche, **dadurch gekennzeichnet, dass**
das Behandeln des Produktgemisches mit Ethanol gemäß Schritt h) bei Temperaturen im Bereich von 40 bis 75 °C durchgeführt wird.

4. Verfahren nach einem der vorangehenden Ansprüche, **dadurch gekennzeichnet, dass**
das in Schritt h) Ethanol in einer Menge im Bereich von 40 bis 60 Gewichtsprozent, bezogen auf das Gesamtgewicht aus dem Ethanol und dem Wasser bzw. dem wässrigen System, zugegeben wird.

5. Verfahren nach einem der vorangehenden Ansprüche, **dadurch gekennzeichnet, dass**
das extrahierende Behandeln gemäß Schritt e) sich über eine Zeitdauer von maximal 4 Stunden, insbesondere eine Zeitdauer von maximal 3 Stunden, erstreckt.

6. Verfahren nach einem der Ansprüche 1 bis 5, **dadurch gekennzeichnet, dass** die Filtration gemäß Schritt i) mit einem Filter mit einer Maschenweite im Bereich von 0,25 µm bis 1,0 µm, insbesondere im Bereich von 0,35 µm bis 0,55 µm, durchgeführt wird.

7. Verfahren zur Herstellung eines Extrakts enthaltend Gallussäure, umfassend die Schritte
1) Zurverfügungstellung von, insbesondere zerkleinerten und/oder, insbesondere und, getrockneten, Pflanzen oder Pflanzenbestandteilen, enthaltend Tannine, insbesondere Tanninsäure, wobei die zur Verfügung gestellte Pflanze ein Sumachgewächs umfasst oder darstellt und wobei die Pflanzenbestandteile Blätter, Hölzer, Rinde, Samen, Wurzeln und/oder Früchte von Sumachgewächsen umfassen oder darstellen,
2) Zurverfügungstellung des Enzyms Tannase,
3) Zurverfügungstellung von Ethanol,
4) gegebenenfalls Zerkleinern bzw. weiteres Zerkleinern der Pflanzen oder Pflanzenbestandteile gemäß Schritt 1),
5) extrahierendes Behandeln der, insbesondere zerkleinerten, Pflanzen oder Pflanzenbestandteile gemäß Schritt 1) und/oder 4), insbesondere oder, mit einem Gemisch enthaltend Ethanol und Wasser unter Erhalt eines Produktgemisches, enthaltend Polyphenole,
6) Abtrennen der, insbesondere zerkleinerten, Pflanzen oder Pflanzenbestandteile aus dem Produktgemisch gemäß Schritt 5) unter Erhalt eines Flüssigextrakts enthaltend die Polyphenole,
7) Entfernen des Ethanols aus dem Flüssigextrakt gemäß Schritt 6) unter Erhalt eines wässrigen Extrakts enthaltend die Polyphenole,
8) Behandeln des wässrigen Extrakts gemäß Schritt 7) mit dem Enzym Tannase unter Erhalt eines wässrigen Systems, enthaltend Gallussäure,
9) evaporatives Konzentrieren des wässrigen Systems, enthaltend Gallussäure, gemäß Schritt 8) unter Erhalt eines wässrigen Konzentrats,
wobei in Schritt 5) Ethanol in einer Menge im Bereich von 60 bis 90 Gewichtsprozent, bezogen auf das Gesamtgewicht aus Ethanol und dem Wasser, zugegeben wird.

8. Verfahren nach einem der Ansprüche 1 bis 7, **dadurch gekennzeichnet, dass** das Abtrennen in Schritt f) oder in Schritt 6) mittels eines Siebs durchgeführt wird.

9. Verfahren nach einem der vorangehenden Ansprüche, **dadurch**
**gekennzeichnet, dass**
die, insbesondere gemahlenen, vorzugsweise zerkleinerten, Pflanzen oder Pflanzenbestandteile in Schritt e) bzw. Schritt 5) immobilisiert, insbesondere in einem Siebträger immobilisiert, vorliegen.

10. Verfahren nach einem der vorangehenden Ansprüche, **dadurch**
**gekennzeichnet, dass**
das extrahierende Behandeln gemäß Schritt e) bzw. Schritt 5) ein Perkolationsverfahren umfasst.

11. Verfahren nach einem der vorangehenden Ansprüche, **dadurch**
**gekennzeichnet, dass**
die Menge an Tannase in Schritt e) bzw. Schritt 8) im Bereich von 0,1 U/g bis 100,0 U/g, bevorzugt im Bereich von 0,2 U/g bis 50,0 U/g, bezogen auf die Menge an Pflanzen oder Pflanzenbestandteilen, liegt.

12. Verfahren nach einem der vorangehenden Ansprüche, **dadurch gekennzeichnet, dass** das wässrige Konzentrat, erhalten durch evaporatives Konzentrieren des nach Schritt k) erhaltenen wässrigen Extrakts, enthaltend Gallussäure, bzw. des wässrigen Systems, enthaltend Gallussäure, gemäß Schritt 9), ein Spissumextrakt darstellt.

13. Verfahren nach Anspruch 12 **dadurch gekennzeichnet, dass** das wässrige Konzentrat einen Brix-Wert im Bereich von 1° bis 100 ° Brix, bevorzugt im Bereich von 10° bis 70° Brix und besonders bevorzugt im Bereich von 20° bis 50° Brix, aufweist.

14. Verfahren nach einem der Ansprüche 7 bis 13, **dadurch gekennzeichnet, dass** das extrahierende Behandeln gemäß Schritt 5) bei Temperaturen im Bereich von 50 bis 70 °C, insbesondere im Bereich von 55 bis 65 °C, durchgeführt wird.

15. Verfahren nach einem der vorangehenden Ansprüche, **dadurch gekennzeichnet, dass**
das extrahierende Behandeln gemäß Schritt e) bzw. das Behandeln des wässrigen Extrakts gemäß Schritt 8) mit dem Enzym Tannase bei Temperaturen im Bereich von 35 bis 40 °C.

16. Verfahren nach einem der Ansprüche 7 bis 15, **dadurch gekennzeichnet, dass** in Schritt 5) Ethanol in einer Menge im Bereich von 70 bis 85 Gewichtsprozent, bezogen auf das Gesamtgewicht aus Ethanol und dem Wasser, zugegeben wird.

17. Verfahren nach einem der vorangehenden Ansprüche, **dadurch gekennzeichnet, dass**
die zur Verfügung gestellte Pflanze Gewürzsumach umfasst oder darstellt und/oder dass die Pflanzenbestandteile Blätter, Rinde und/oder Früchte, insbesondere Früchte, von Gewürzsumach umfassen oder darstellen.

18. Wässriges Konzentrat, enthaltend Gallussäure, erhalten gemäß einem Verfahren nach einem der vorangehenden Ansprüche, **gekennzeichnet durch** eine Trockenmasse im Bereich von 5 bis 80 Gewichtsprozent, bevorzugt im Bereich von 10 bis 50 Gewichtsprozent und besonders bevorzugt im Bereich von 15 bis 40 Gewichtsprozent, jeweils bezogen auf das Gesamtgewicht des wässrigen Konzentrats, durch einen pH-Wert im Bereich von 1,5 bis 5,5, bevorzugt im Bereich von 1,75 bis 4,5 und besonders bevorzugt im Bereich von 2,0 bis 4,0, durch eine Menge an Gallussäure von mindestens 10 g/kg Trockenmasse, bevorzugt mindestens 50 g/kg Trockenmasse und besonders bevorzugt mindestens 75 g/kg Trockenmasse, und/oder eine Menge an Gallussäure im Bereich von 10 bis 250 g/kg Trockenmasse, bevorzugt im Bereich von 50 bis 200 g/kg Trockenmasse und besonders bevorzugt im Bereich von 75 bis 175 g/kg Trockenmasse, und durch eine relative Dichte [20/20] im Bereich von 1,001 bis 1,400, bevorzugt im Bereich von 1,0100 bis 1,2500, ermittelt mittels Biegeschwinger-Verfahren nach dem in der Beschreibung genannten Verfahren, wobei das wässrige Konzentrat eine Trolox-äquivalente antioxidative Kapazität im Bereich von 500 bis 6000 µmol/g, bezogen auf die Trockenmasse des Extrakts, aufweist, bestimmt mit dem DPPH (2,2-diphenyl-1-picrylhydrazyl)-Assay nach dem in der Beschreiung genannten Bestimmungsverfahren.

19. Lebensmittel, insbesondere Getränk, oder Nahrungsergänzungsmittel, enthaltend oder gebildet aus
einem wässrigen Konzentrat gemäß Anspruch 18.

## Claims

1. Method for producing an extract containing gallic acid, comprising the steps
a) providing, in particular dried and/or, in particular and, comminuted, plants or plant components containing tannins, in particular tannic acid, wherein the provided plant comprises or is a sumac plant and wherein the plant components comprise or are leaves, wood, bark, seeds, roots and/or fruits of sumac plants,
b) providing the enzyme tannase,
c) providing ethanol,
d) optionally comminuting or further comminuting, respectively, of the plants or plant components according to step a),
e) extracting the, in particular comminuted, plants or plant components obtained according to step a) and/or d), in particular or, with water in the presence of the enzyme tannase to obtain a product mixture containing gallic acid,
f) separating the, in particular comminuted, plants or plant components from the product mixture obtained according to step e), in particular following step e), and
g) evaporative concentrating of the product mixture containing gallic acid obtained according to step e) or f), in particular following step e) or f) and/or before step h), and
h) treating the product mixture according to step e) or f) with ethanol and treating the product mixture according to step e) or f) or g) with heat for the deactivation of the enzyme tannase and for the denaturing and precipitation of proteins and/or polysaccharides, and
i) filtering the treated product mixture according to step h), and
j) removing the ethanol added in step h) from the treated product mixture according to step h) or the filtered product mixture according to step i),
k) evaporative concentrating of the aqueous extract containing gallic acid obtained according to step j) to obtain an aqueous concentrate,
wherein ethanol is added in step h) in an amount in the range from 20 to 65 percent by weight, based on the total weight of the ethanol and the water or the aqueous system, respectively.

2. Method according to claim 1, **characterized in that**
the extractive treatment according to step e) is carried out at temperatures in the range from 20 to 45°C.

3. Method according to one of the preceding claims, **characterized in that**
the treatment of the product mixture with ethanol according to step h) is carried out at temperatures in the range from 40 to 75°C.

4. Method according to one of the preceding claims, **characterized in that**
the ethanol is added in step h) in an amount in the range from 40 to 60 percent by weight, based on the total weight of the ethanol and the water or the aqueous system, respectively.

5. Method according to one of the preceding claims, **characterized in that**
the extractive treatment according to step e) extends over a period of a maximum of 4 hours, in particular a period of a maximum of 3 hours.

6. Method according to one of claims 1 to 5, **characterized in that**
the filtration according to step i) is carried out with a filter having a mesh width in the range from 0.25 µm to 1.0 µm, in particular in the range from 0.35 µm to 0.55 µm.

7. Method for producing an extract containing gallic acid, comprising the steps
1) providing, in particular comminuted and/or, in particular and, dried, plants or plant components containing tannins, in particular tannic acid, wherein the provided plant comprises or is a sumac plant and wherein the plant components comprise or are leaves, wood, bark, seeds, roots and/or fruits of sumac plants,
2) providing the enzyme tannase,
3) providing ethanol,
4) optionally comminuting or further comminuting, respectively, the plants or plant components according to step 1),
5) extractive treatment of the, in particular comminuted, plants or plant components according to step 1) and/or 4), in particular or, with a mixture containing ethanol and water to obtain a product mixture containing polyphenols,
6) separating the, in particular comminuted, plants or plant components from the product mixture according to step 5) to obtain a liquid extract containing the polyphenols,
7) removing the ethanol from the liquid extract according to step 6) to obtain an aqueous extract containing the polyphenols,
8) treating the aqueous extract according to step 7) with the enzyme tannase to obtain an aqueous system containing gallic acid,
9) evaporative concentrating of the aqueous system containing gallic acid according to step 8) to obtain an aqueous concentrate,
wherein ethanol is added in step 5) in an amount in the range from 60 to 90 percent by weight, based on the total weight of ethanol and the water.

8. Method according to one of claims 1 to 7, **characterized in that**
the separation in step f) or in step 6) is carried out by means of a sieve.

9. Method according to one of the preceding claims, **characterized in that**
the, in particular ground, preferably comminuted, plants or plant components are in step e) or step 5), respectively, are present immobilized, in particular immobilized in a portafilter.

10. Method according to one of the preceding claims, **characterized in that**
the extractive treatment according to step e) or step 5), respectively, comprises a percolation method.

11. Method according to one of the preceding claims, **characterized in that**
the amount of tannase in step e) or step 8), respectively is in the range from 0.1 U/g to 100.0 U/g, preferably in the range from 0.2 U/g to 50.0 U/g, based on the amount of plants or plant components.

12. Method according to one of the preceding claims, **characterized in that** the aqueous concentrate obtained by evaporative concentrating of the aqueous extract containing gallic acid obtained according to step k), or of the aqueous system containing gallic acid, according to step 9), respectively, represents a spissum extract.

13. Method according to claim 12, **characterized in that**
the aqueous concentrate has a Brix value in the range from 1° to 100° Brix, preferably in the range from 10° to 70° Brix and particularly preferably in the range from 20° to 50° Brix.

14. Method according to one of claims 7 to 13, **characterized in that**
the extractive treatment according to step 5) is carried out at temperatures in the range from 50 to 70°C, in particular in the range from 55 to 65°C.

15. Method according to one of the preceding claims, **characterized in that**
the extractive treatment according to step e) or the treatment of the aqueous extract according to step 8) with the enzyme tannase, respectively, is carried out at temperatures in the range from 35 to 40°C.

16. Method according to one of claims 7 to 15, **characterized in that**
ethanol is added in step 5) in an amount in the range from 70 to 85 percent by weight, based on the total weight of ethanol and the water.

17. Method according to one of the preceding claims, **characterized in that**
the provided plant comprises or is fragrant sumac and/or that the plant components comprise or are leaves, bark and/or fruits, in particular fruits, of fragrant sumac.

18. Aqueous concentrate containing gallic acid, obtained according to a method according to one of the preceding claims, **characterized by** a dry mass in the range from 5 to 80 percent by weight, preferably in the range from 10 to 50 percent by weight and particularly preferably in the range from 15 to 40 percent by weight, in each case based on the total weight of the aqueous concentrate, by a pH value in the range from 1.5 to 5.5, preferably in the range from 1.75 to 4.5 and particularly preferably in the range from 2.0 to 4.0, by an amount of gallic acid of at least 10 g/kg dry mass, preferably at least 50 g/kg dry mass and particularly preferably at least 75 g/kg dry mass, and/or an amount of gallic acid in the range from 10 to 250 g/kg dry mass, preferably in the range from 50 to 200 g/kg dry mass and particularly preferably in the range from 75 to 175 g/kg dry mass, and by a relative density [20/20] in the range from 1.001 to 1.400, preferably in the range from 1.0100 to 1.2500, determined by way of the oscillating u-shape method according to the method mentioned in the description, wherein the aqueous concentrate has a Trolox-equivalent antioxidant capacity in the range from 500 to 6000 µmol/g, based on the dry mass of the extract, determined with the DPPH (2.2-diphenyl-1-picrylhydrazyl) assay according to the determination method mentioned in the description.

19. Foodstuff, in particular beverage, or dietary supplement, containing or formed from an aqueous concentrate according to claim 18.

## Revendications

1. Procédé de préparation d'un extrait contenant de l'acide gallique, comprenant les étapes
a) mise à disposition de plantes ou de constituants de plante, en particulier séchés et/ou, en particulier et, broyés, contenant des tanins, en particulier de l'acide tannique, la plante mise à disposition comprenant ou représentant une plante de sumac et les constituants de plante comprenant ou représentant des feuilles, des bois, des écorces, des graines, des racines et/ou des fruits de plantes de sumac,
b) mise à disposition de l'enzyme tannase,
c) mise à disposition d'éthanol,
d) le cas échéant broyage ou broyage supplémentaire des plantes ou des constituants de plante selon l'étape a),
e) traitement par extraction des plantes ou des constituants de plante, en particulier broyés, obtenus selon l'étape a) et/ou d), en particulier ou, avec de l'eau en présence de l'enzyme tannase avec obtention d'un mélange de produits contenant de l'acide gallique,
f) séparation des plantes ou des constituants de plante, en particulier broyés, du mélange de produits obtenu selon l'étape e), en particulier à la suite de l'étape e), et
g) concentration par évaporation du mélange de produits contenant de l'acide gallique, obtenu selon l'étape e) ou f), en particulier à la suite de l'étape e) ou f) et/ou avant l'étape h), et
h) traitement du mélange de produits selon l'étape e) ou f) avec de l'éthanol et traitement du mélange de produits selon l'étape e) ou f) ou g) avec de la chaleur pour la désactivation de l'enzyme tannase et pour la dénaturation et la précipitation de protéines et/ou de polysaccharides et
i) filtration du mélange de produits traité selon l'étape h) et
j) élimination de l'éthanol ajouté à l'étape h) du mélange de produits traité selon l'étape h) ou du mélange de produits filtré selon l'étape i),
k) concentration par évaporation de l'extrait aqueux contenant de l'acide gallique, obtenu selon l'étape j) avec obtention d'un concentré aqueux,
où de l'éthanol étant ajouté à l'étape h) en une quantité dans la plage de 20 à 65 pour cent en poids, par rapport au poids total de l'éthanol et de l'eau ou du système aqueux.

2. Procédé selon la revendication 1, **caractérisé en ce que**
le traitement par extraction selon l'étape e) est réalisé à des températures dans la plage de 20 à 45 °C.

3. Procédé selon l'une quelconque des revendications précédentes, **caractérisé en ce que**
le traitement du mélange de produits avec de l'éthanol selon l'étape h) est réalisé à des températures dans la plage de 40 à 75 °C.

4. Procédé selon l'une quelconque des revendications précédentes, **caractérisé en ce que**
l'éthanol est ajouté à l'étape h) en une quantité dans la plage de 40 à 60 pour cent en poids, par rapport au poids total de l'éthanol et de l'eau ou du système aqueux.

5. Procédé selon l'une quelconque des revendications précédentes, **caractérisé en ce que**
le traitement par extraction selon l'étape e) s'étend sur une durée de 4 heures au maximum, en particulier une durée de 3 heures au maximum.

6. Procédé selon l'une quelconque des revendications 1 à 5, **caractérisé en ce que** la filtration selon l'étape i) est réalisée avec un filtre ayant une largeur de maille dans la plage de 0,25 µm à 1,0 µm, en particulier dans la plage de 0,35 µm à 0,55 µm.

7. Procédé de préparation d'un extrait contenant de l'acide gallique, comprenant les étapes
1) mise à disposition de plantes ou de constituants de plante, en particulier broyés et/ou, en particulier et, séchés, contenant des tanins, en particulier de l'acide tannique, la plante mise à disposition comprenant ou représentant une plante de sumac et les constituants de plante comprenant ou représentant des feuilles, des bois, des écorces, des graines, des racines et/ou des fruits de plantes de sumac,
2) mise à disposition de l'enzyme tannase,
3) mise à disposition d'éthanol,
4) le cas échéant broyage ou broyage supplémentaire des plantes ou des constituants de plante selon l'étape 1),
5) traitement par extraction des plantes ou des constituants de plante, en particulier broyés, selon l'étape 1) et/ou 4), en particulier ou, avec un mélange contenant de l'éthanol et de l'eau avec obtention d'un mélange de produits contenant des polyphénols,
6) séparation des plantes ou des constituants de plante, en particulier broyés, du mélange de produits selon l'étape 5) avec obtention d'un extrait liquide contenant les polyphénols,
7) élimination de l'éthanol de l'extrait liquide selon l'étape 6) avec obtention d'un extrait aqueux contenant les polyphénols,
8) traitement de l'extrait aqueux selon l'étape 7) avec l'enzyme tannase avec obtention d'un système aqueux contenant de l'acide gallique,
9) concentration par évaporation du système aqueux contenant de l'acide gallique selon l'étape 8) avec obtention d'un concentré aqueux,
où de l'éthanol étant ajouté à l'étape 5) en une quantité dans la plage de 60 à 90 pour cent en poids, par rapport au poids total de l'éthanol et de l'eau.

8. Procédé selon l'une quelconque des revendications 1 à 7, **caractérisé en ce que** la séparation à l'étape f) ou à l'étape 6) est réalisée au moyen d'un tamis.

9. Procédé selon l'une quelconque des revendications précédentes, **caractérisé en ce que**
les plantes ou les constituants de plante, en particulier broyés, de préférence broyés, sont immobilisés à l'étape e) ou à l'étape 5), en particulier immobilisés dans un support de tamis.

10. Procédé selon l'une quelconque des revendications précédentes, **caractérisé en ce que**
le traitement par extraction selon l'étape e) ou l'étape 5) comprend un procédé de percolation.

11. Procédé selon l'une quelconque des revendications précédentes, **caractérisé en ce que**
la quantité de tannase à l'étape e) ou à l'étape 8) se situe dans la plage de 0,1 U/g à 100,0 U/g, de préférence dans la plage de 0,2 U/g à 50,0 U/g, par rapport à la quantité de plantes ou de constituants de plante.

12. Procédé selon l'une quelconque des revendications précédentes, **caractérisé en ce que** le concentré aqueux, obtenu par concentration par évaporation de l'extrait aqueux contenant de l'acide gallique, obtenu selon l'étape k), ou du système aqueux contenant de l'acide gallique, selon l'étape 9), est un extrait de spissum.

13. Procédé selon la revendication 12, **caractérisé en ce que**
le concentré aqueux présente une valeur Brix dans la plage de 1° à 100° Brix, de préférence dans la plage de 10° à 70° Brix et de manière particulièrement préférée dans la plage de 20° à 50° Brix.

14. Procédé selon l'une quelconque des revendications 7 à 13, **caractérisé en ce que** le traitement par extraction selon l'étape 5) est réalisé à des températures dans la plage de 50 à 70 °C, en particulier dans la plage de 55 à 65 °C.

15. Procédé selon l'une quelconque des revendications précédentes, **caractérisé en ce que**
le traitement par extraction selon l'étape e) ou le traitement de l'extrait aqueux selon l'étape 8) avec l'enzyme tannase est réalisé à des températures dans la plage de 35 à 40 °C.

16. Procédé selon l'une quelconque des revendications 7 à 15, **caractérisé en ce que** de l'éthanol est ajouté à l'étape 5) en une quantité dans la plage de 70 à 85 pour cent en poids, par rapport au poids total de l'éthanol et de l'eau.

17. Procédé selon l'une quelconque des revendications précédentes, **caractérisé en ce que**
la plante mise à disposition comprend ou représente du sumac d'épicße et/ou **en ce que** les constituants de plante comprennent ou représentent des feuilles, des écorces et/ou des fruits, en particulier des fruits, du sumac d'épicé.

18. Concentré aqueux contenant de l'acide gallique, obtenu selon un procédé selon l'une quelconque des revendications précédentes, **caractérisé par** une masse sèche dans la plage de 5 à 80 pour cent en poids, de préférence dans la plage de 10 à 50 pour cent en poids et de manière particulièrement préférée dans la plage de 15 à 40 pour cent en poids, à chaque fois par rapport au poids total du concentré aqueux, par une valeur de pH dans la plage de 1,5 à 5,5, de préférence dans la plage de 1,75 à 4,5 et de manière particulièrement préférée dans la plage de 2,0 à 4,0, par une quantité d'acide gallique d'au moins 10 g/kg de masse sèche, de préférence d'au moins 50 g/kg de masse sèche et de manière particulièrement préférée d'au moins 75 g/kg de masse sèche, et/ou une quantité d'acide gallique dans la plage de 10 à 250 g/kg de masse sèche, de préférence dans la plage de 50 à 200 g/kg de masse sèche et de manière particulièrement préférée dans la plage de 75 à 175 g/kg de masse sèche, et par une densité relative [20/20] dans la plage de 1,001 à 1,400, de préférence dans la plage de 1,0100 à 1,2500, déterminée au moyen d'un procédé à oscillateur de flexion selon le procédé mentionné dans la description, le concentré aqueux présentant une capacité antioxydante équivalente à Trolox dans la plage de 500 à 6000 µmol/g, par rapport à la masse sèche de l'extrait, déterminée avec le dosage DPPH (2,2-diphényl-1-picrylhydrazyl) selon le procédé de détermination mentionné dans la description.

19. Aliment, en particulier boisson, ou complément alimentaire, contenant ou formé à partir
d'un concentré aqueux selon la revendication 18.
